Europäisches Patentamt

European Patent Office

Office européen des brevets

(19)

(11) Publication number: **0 004 760**
A2

## EUROPEAN PATENT APPLICATION

(12)

(21) Application number: 79300534.9

(22) Date of filing: 30.03.79

(51) Int. Cl.²: **G 01 N 33/16**
**A 61 B 5/14**

(30) Priority: 05.04.78 GB 1329378

(43) Date of publication of application:
17.10.79 Bulletin 79/21

(84) Designated Contracting States:
BE CH DE FR IT LU NL SE

(71) Applicant: **SIRA INSTITUTE LIMITED**
**South Hill**
**Chislehurst Kent BR7 5EH(GB)**

(84) Designated Contracting States:
BE CH DE FR IT LU NL SE

(71) Applicant: **SAINT PETERS'S RESEARCH TRUST**
**LIMITED**
**172-176 Shaftesbury Avenue**
**London WC2H 8JE(GB)**

(84) Designated Contracting States:
**BE CH DE FR IT LU NL SE**

(72) Inventor: **Simpson, Roger Jeremy**
**2 Cross Keys Cottage Ashgrove Road**
**Sevenoaks Kent(GB)**

(72) Inventor: **Gawthorpe, Janet Ann**
**20 The Chenies Petts Wood**
**Orpington Kent(GB)**

(72) Inventor: **Lawrence, Charles Alexander**
**121 Latymer Court Hammersmith Road**
**London W6(GB)**

(74) Representative: **Wright, Hugh Ronald**
**Brookes & Martin, 52/54 High Holborn**
**London WC1V 6SE(GB)**

(54) Apparatus for determining the presence of predetermined constituents in a liquid.

(57) Apparatus for determining the presence of predetermined constituents such as potassium ions, calcium ions, or gases in a liquid such as blood, beer, milk or effluent, in which the liquid is passed through a helical passage partly defined by membrane means through which the desired constituents may pass. The desired constituents which pass through the membrane are subsequently analysed by analytical means which is thereby not contaminated by other constituents of the liquid under test.

Fig. 2

0004760

The present invention relates to apparatus for analysing a liquid such as blood, milk, beer and effluent.

There are generally two ways of analysing a liquid for its various constituents. The first of these is a non-continuous sampling method in which a volume of the liquid is taken and is then analysed for the various constituents under consideration.

The second method is a continuous analysis method in which all or part of the liquid is allowed to pass through an analytical apparatus.

An advantage of the first method is its accuracy but two serious disadvantages are the time taken for the test and the quantity of the liquid that must be removed and cannot thereafter be replaced in the bulk of the liquid. An advantage of the second method is that the liquid which is used for sampling is flowing continuously and therefore it takes into account variations in the constituents as they occur. Also the liquid after analysis is usually returned to the bulk of the liquid. However, a problem is that the analytical apparatus contacts the liquid and, particularly if the liquid is blood, there is a risk of contamination of any ion selective electrodes/in or other measuring devices the apparatus by various constituents, such as heavy protein molecules, of the blood.

0004760

There is therefore a desire for the continuous analysis of a liquid such as blood which is safe and will not contaminate the analysing means so that the variations in the levels of the various constituents of the blood may be continuously recorded and the blood after analysis may be returned to the patient's body.

The invention provides apparatus for determining the presence of predetermined constituents in a liquid including a helical passage for the liquid, membrane means forming a wall of said helical passage permeable to said predetermined constituents to provide predetermined relative concentrations of said constituents on each side of the membrane and means for analysing a carrier fluid containing the predetermined constituents which have permeated through the membrane.

The carrier fluid may be a separate fluid and may be a gas or a liquid. The carrier fluid may alternatively be water separated from the liquid under test.

There may be provided analysis means for the analysis of dissolved ions in the carrier fluid, said analysis means including electrodes which are sensitive to the particular constituent in question and a reference electrode.

0004760

The apparatus is particularly applicable to this arrangement since some constituents of the liquid under test may affect the electrodes but the present apparatus allows those contaminants to remain in the liquid under test and not to be passed through the membrane.

In a particular preferred arrangement for determining the presence of predetermined constituents in blood, means may be provided for connecting the apparatus to a patient whereby blood may be continuously withdrawn from a patient, passed through the apparatus and thereafter returned to the patient.

Preferred embodiments of the invention will now be described by way of example only and with reference to the accompanying drawings in which,

Figure 1 is a diagrammatic representation of a first embodiment of apparatus according to the invention,

Figure 2 is a side view, partly cut away, of a preferred cell for use in apparatus of the invention,

Figure 3 is a vertical section on the line 3-3 of Figure 2,

Figure 4 shows a preferred form of the electrolyte analysis unit for use with the apparatus of the invention, and,

Figure 5 is a cross-section through part of the apparatus of Figure 2 showing liquid flow.

Although described here as a blood analyser the apparatus is very suitable to the analysis of other liquids in, for example, fermentation processes, brewing or effluent treatments or in analysis of milk or other dairy products.

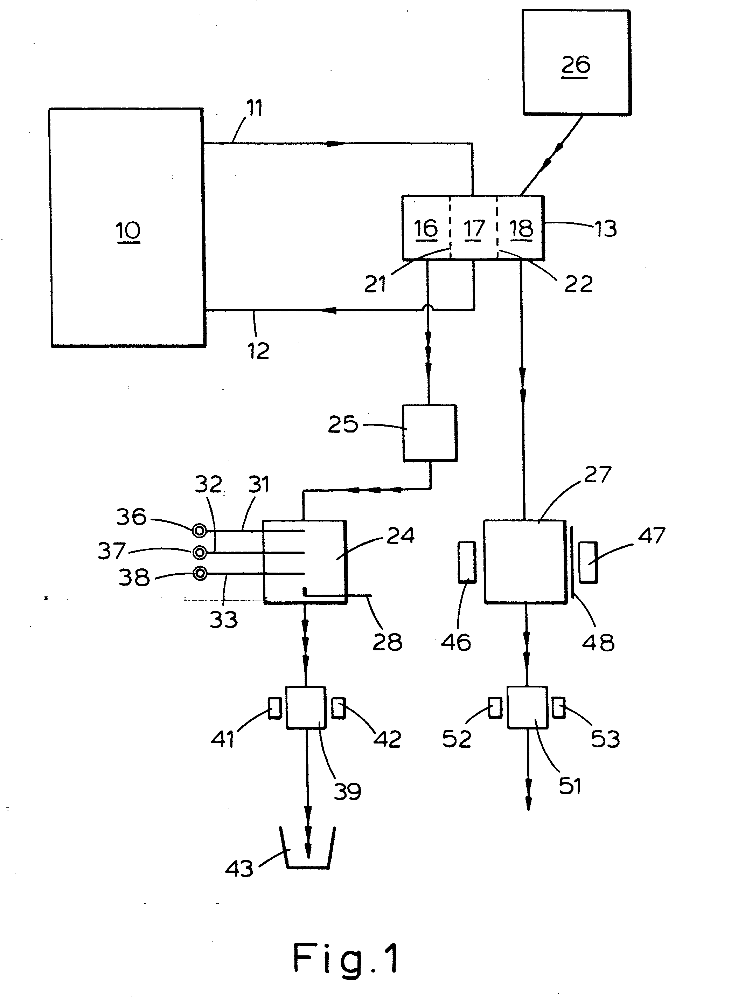

The apparatus of Figure 1 is for the direct analysis of a patient's blood to give the concentration of certain constituents of clinical importance. The apparatus consists of two related but separate systems one for the measurement of electrolytes such as potassium, calcium, fluoride or other ions and the other for the estimation of the partial pressures of dissolved gases such as carbon dioxide, ammonia, oxygen and anaesthetic agents. In practice, only one half need be used at any one time and the apparatus may be simplified for certain uses by only providing for electrolyte or the dissolved gas analysis.

The apparatus to be described is attached externally to the patient 10 and is connected to an artery and a vein. Flexible tubes 11, 12 lead from the patient to the apparatus, blood passing from the patient 10 to the apparatus via tube 11 and from the apparatus back to the patient via tube 12.

The flexible tubes are preferably silicone rubber tubing or other non toxic hypothrombogenic material.

- 6 -

A cell 13 is provided of any desired shape such as to make
it most efficient and most easily cleaned, the cell 13
being divided into three compartments 16, 17, 18 which
are shaped to avoid stagnant regions in which clots might
form. Blood from the patient 10 passes through the tube
11 and through the central compartment 17. The compartments
16 and 17 are divided by a membrane 21 and
compartments 17, 18 are divided by membrane 22
The membrane 21 is of a type commercially available which
allows for the passage only of electrolytes and water and
the membrane 22 is of a type, also commercially available,
which allows for the passage of dissolved gases only. The
arrangement is such as to provide a relatively rapid flow
past the surface of the ion/water permeable membrane to
prevent a build up of macromolecules with resultant
reduction in filtration rate.

The gas porous membrane may be of thin silicone rubber or
of microporous polypropylene (celgard 2500). The ion/water
membrane may be ultrafiltration membranes such as that made
by Amicon or Millipore which is used in artificial
kidneys. The Amicon or Millipore ultrafiltration membranes
have sharply defined molecular weight cut off points above
which they will not permit passage. Membranes with 10,000
and 30,000 MW cut off are preferred.

The compartment 16 is connected to an electrolyte analysis
unit 24 by fine flexible tubes. There is provided a
peristaltic pump 25 between the compartment 16 and the
electrolye analysis unit 24 which can be used to regulate

0004760

the flow of the liquid between the compartment 16 and
the unit 24.

The compartment 18 is connected to a carrier gas supply
26 and the gas passing through the compartment 18 is
passed to a dissolved gas analysis unit 27.

The electrolyte analysis unit 24 consists of a thermostatically controlled cell of small volume into which
are inserted one or more ion selective electrodes 31,
32, 33 responding to ions such as potassium, calcium
or sodium.  Downstream from the electrodes is positioned
the tip of a reference electrode 28.  The potentials of
the electrodes 31, 32, 33 are measured with respect to
the reference electrode 28 using respective high input
impedence millivoltmeters 36, 37, 38.  The meters 36, 37
38 are calibrated non-linearly in concentration.
Alternatively the same high input impedance millivoltmeter
would be used for each electrode in turn, readings of
each parameter being taken every five minutes or so.
Analogue or digital signal processing can be used to
give a direct concentration reading.

On leaving the electrolyte analysis unit 24 the liquid
passes through a small transparent chamber 39.  A light
source 41 and photodetector 42  on the opposite side
are provided to check the transparency of the fluid.
Alternatively, the chamber 39 may be provided before
the unit 24.

- 8 -

0004760

Finally the liquid is allowed to fall in drops into a waste container 43. A light source (not shown) and photodetector may be provided with an electronic timer to give off a warning should the interval between drops of waste liquid become abnormal ie too long indicating membrane blockage or pump failure or too short indicating membrane rupture.

The dissolved gas analyser unit 27 comprises a cell having windows transparent to infra-red radiation in the required regions of the spectrum. There is a source 46 of radiation of a suitable wavelength preferably a black body with suitable filter and a photodetector 47, the photodetector 47 detecting through a suitable filter radiation of a wavelength which is strongly absorbed by the gas to be measured. By selection of the filter 48, with either the same photodetector 47 or different photodetectors, different gases may be analysed.

A similar arrangement for detecting membrane rupture is provided in the form of a small transparent chamber 51, alight source 52, and a photodetector 53 to check the transparency of the gas.

The presence of a suitable gas flow can be detected by a cooling effect on a hot wire forming part of an electrical bridge. The gas passes from the unit 27 to waste through tube 54.

The tubes connecting the various components of the apparatus are all fine bore so as to minimise the quantity of liquid or gas in the system.

In use the tubes 11 and 12 are suitably connected to arteries and veins in the patient 10 and blood flows through the apparatus at approximately 50 to 250 ml/min. The carrier gas supply 26 is connected to the cell 13 and the pump 25 switched on to provide a metered flow of water and ions through the membrane 21, through the compartment 16 to the unit 24. Similarly,gas is passed from the supply 26 through the compartment 18 to the unit 27 and although not shown some metering device may be provided corresponding to the pump 25. Blood thereby passes through the central compartment 17 and back to the patient. Water and ions pass through the membrane 21 and the concentration of the ions in the water reaches an equilibrium dependent on their concentration in the blood. In most circumstances, the concentrations would be identical but under certain circumstances, perhaps depending on flow rate and other variable factors, the concentrations may not be identical but will be of a predetermined proportion. Dissolved gas

0004760

in the blood passes through the membrane 22 into the gas 26 until an equilibrium is set up between the concentration of the dissolved gas in the blood and the concentration of that dissolved gas in the carrier gas.

After passing through the central compartment 17 the blood returns to the patient, the net effect having been that some of the ions, water and dissolved gas in the blood has been removed but since the quantities involved are small this has no noticeable effect on the patient's blood system. Furthermore, the blood only comes into contact with the tubes 11 and 12 and the inside of the compartment 17 and the membranes 21 and 22 all of which are non-toxic hypothrombogenic materials and are easy to maintain clean and sterile. Some components may be disposable.

The liquid from the compartment 16 is controlled to pass at a predetermined rate by the pump 25 (e.g. 50-350$\mu$l/min) which under some circumstances may be drawing fluid from the cell 16 and in other circumstances may be slowing down the flow. The liquid passes to the analysis unit 24. After equilibrium has been obtained the meters 36,37,38 indicate the concentrations of the various electrolytes. The liquid passes from the unit 24 to the chamber 39. In the event of the membrane 21

- 11 -

rupturing, blood would pass into the carrier liquid 0004760
and would colour the liquid passing through the
transparent chamber 39 so that the light would be
cut off from the detector 42.   The peristaltic pump 25
would them automatically be switched off effectively
cutting off the flow and a warning given.

After leaving chamber 39, the liquid passes away
as described.

The carrier gas from the compartment 18 continuously
passes to the analysis unit 27.   After equilibrium is
obtained so that the relative partial pressures of the gas
in the blood and in the carrier gas are known, the analyser
unit 27 is operated.   Carbon dioxide absorbs light of a
specific wavelength and thus by measuring the amount
of light of that wavelength by means of the cell 47 the
amount of carbon dioxide in the carrier gas can be
readily measured.   In a simpler apparatus $CO_2$ may be
measured using an electrode.

The concentration of oxygen in the carrier gas can
be measured by a conventional analyser or other means.

As before the presence of blood in the gas stream which
would indicate that the membrane 22 could have ruptured
could be checked by the chamber 51.

0004760

The apparatus can be used to simply provide a qualitative analysis of the blood. For example, it can be utilised to measure whether or not there are ions or gas of a predetermined type present in the blood. Alternatively, it is often necessary to know in a clinical situation when the concentration of a particular ion or gas increases or decreases and the apparatus can then simply be set up and variations in the level of the particular gas or ion under consideration noted.

If however it is required to provide a quantitative measure of the ion and concentration of gas and dissolved gas concentration in the blood, then there are several ways of calibrating the apparatus. In the first of these, before or after analysis, in place of the blood being provided, a predetermined known blood sample may be passed through the compartment 17 with a known level of ion concentration and gas concentrations and the readings of various meters noted.

A second way of calibrating apparatus is to collect the waste gas and waste liquid and to analyse them on a batch basis according to a conventional method. These may then provide an indication of the levels of the dissolved gas and ions in the blood and their relationship to the indications of the various meters. Thirdly, one may provide standard solutions in place of the filtrate and analyse them with the analysing apparatus. Fourthly, one

0004760

may analyse effluent liquid leaving electrode cell, or take independent blood samples from the patient for check analysis.

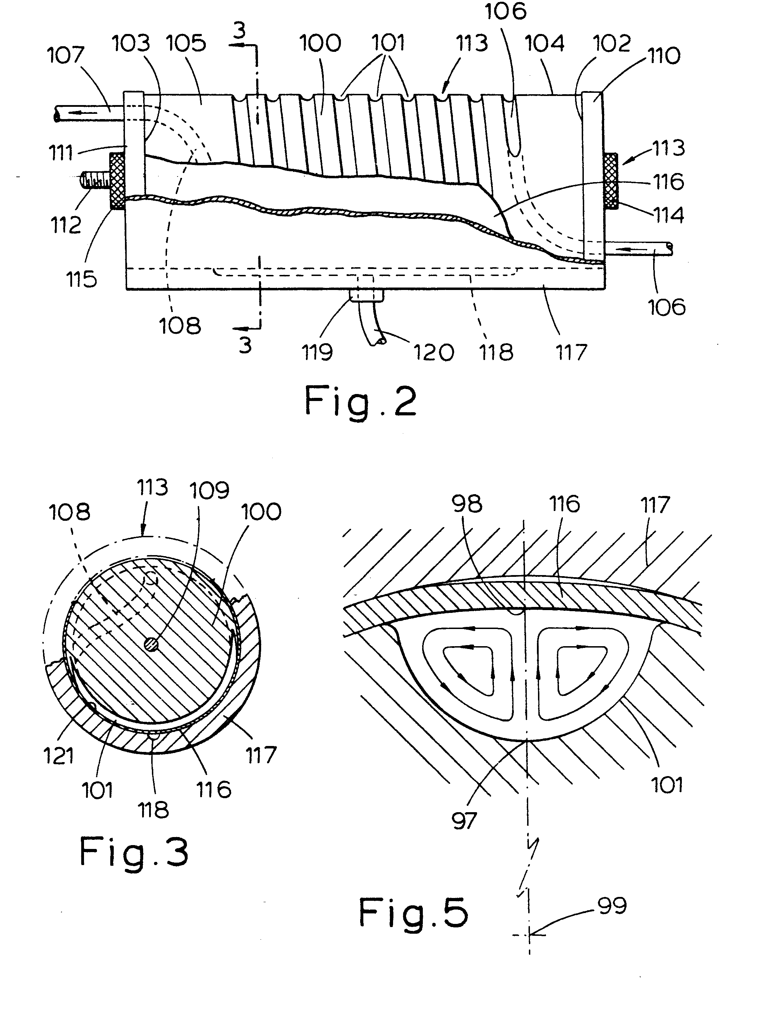

A preferred form of cell corresponding to the cell 13 of Figure 1 is illustrated in Figures 2 and 3. The preferred cell 113 comprises a generally cylindrical core 100 of medical grade silicone rubber. Moulded around the outer periphery of the core 100 is a helical groove 101 being of generally semi-circular cross section and having a pitch (4 mm) approximately equal to the width (2.5 mm) of the groove. The groove 101 does not continue to opposite ends 102, 103 of the core 100 so that a smooth cylindrical land 104, 105 is provided adjacent each end 102, 103.

A plastic tube 106 of silicone rubber is moulded into the core 100 so as to interconnect with the end of the helical groove 101 adjacent the end 102 and as is clear from the Figure the tube 106 is disposed adjacent the groove 101 to continue a helical path corresponding to the path of the groove 101 and then passes into the interior of the core 100 and passes out of the core 100 at the end 102 in a direction parallel to the axis of the core 100. The path of the tube 106 within the core is shown in dotted lines in Figure 2. A similar tube is arranged at the end of 103 of the core 100 to interconnect with the opposite end of the helical groove 101 and the path of this tube 107 is shown in dotted lines indicated by the reference numeral 108 in both Figures 2 and 3. In this way

- 14 -                    0004760

a smooth path is provided for the blood being passed from the inlet tube 106 through to the helical groove 101 and out through the outlet tube 107. The inlet tube 106 corresponds with the tube 11 of Figure 1 and the outlet tube 107 corresponds with the tube 12 of Figure 1.

An axial bore 109 is provided through the core 100 and there are provided adjacent each end 102, 103 of the core 100 circular metal plates 110, 111 respectively. These metal plates include bores therethrough so as to allow free passage of the inlet tube 106 and 107 respectively and also a central bore corresponding to the bore 109. The shank 112 of a screw 113 having a head 114 passes through the axial bore 109 and at the other end a knurled nut 115 is screwed on to the shank 112 whereby the two circular plates 110, 111 and core 100 may be clamped together. As will be clear from Figure 2 the diameter of the core 100 and of the circular plates 110, 111 is the same when the core 100 is in an unstressed condition.

A membrane 116 curved to form a cylinder is provided to surround the core 100 and this membrane may be of the type described with reference to Figure 1 such as an ion permeable membrane or a gas permeable membrane as desired.

Surrounding the core 100 and the membrane 116 is a hollow perspex cylinder 117 the diameter of the inner surface 121 of which is only marginally greater than the external diameter of the core 100 and the membrane 116. The axial length of the cylinder 117 corresponds to the overall axial length of the core 100 and the two plates 110, 111. The inner surface 121 of the cylinder 117 includes a semi circular groove 118 which extends generally parallel to the axis from adjacent one end of the cylinder to adjacent the opposite end. It does not however reach either end. The groove 118 communicates with an outlet 120 via a radial bore 119 through the cylinder 117. Other grooves in the inner surface 121 could be provided draining into groove 118.

In use the apparatus is cleaned in its dissembled state and is then assembled so that the core 100 is lightly clamped by means of the screw 113 between the plates 110, 111. The cylindrical membrane 116 is then passed over the core 100 and the assembly inserted into the cylinder 117. There is a clearance between the inner surface 121 of the cylinder 117 and the outside surface of the membrane 116. The nut 115 is then tightened on to the shank 112 to thereby compress the core 100 between the circular plates 110, 111. The effect of this is to expand the diameter of the core 100 until it is rigidly mounted within the cylinder 122. A seal is formed between the core 100, the membrane 116, and between the membrane 116 and the cylinder 117. There is unlikely to be leakage between the successive turns of the groove 101 but if there is this is of no immediate concern and the provision of the lands 104, 105 provides a good seal at the axial ends of the cell.

Blood from the patient is then passed between the inlet tube 106 and the outlet tube 107 passing between these two tubes along the length of the helical groove 101. In passing along the helical groove 101, ions or gas (depending upon which membrane 116 is utilised) will pass through the membrane 116 where it faces the helical groove 101.

Where the membrane 116 faces the helical groove 101 it is not clamped between two opposing surfaces and is therefore slightly spaced from the cylinder 117. Gases or ions (depending upon which membrane is utilised) will pass through the membrane 116 where it faces the helical groove 101 and will pass along a path between the membrane 116 and the inner surface 121 of the cylinder 117 corresponding to the helical groove 101 but outside it until it reaches the groove 118 and thence to the outlet tube 120.

The arrangement described has particular advantages. As was described with reference to Figure 1 one of the major problems in dealing with blood is the possibility of clotting and macromolecular blockage of the membrane, unless these molecules are constantly washed off. In the present instance there is a very low rate of flow of blood and clotting and macromolecular deposition is a severe problem. The present inventors have realised that clotting and macromolecular deposition can be reduced by eliminating as far as possible laminar flow adjacent the membrane. Laminar flow, which is particularly likely to occur in straight tube, is the phenomenon whereby layers of the liquid adjacent the walls of a tube or groove will tend to move either very slowly or remain substantially stationary. This encourages clotting and deposition of large molecules.

The use of a helical path is particularly advantageous since according to fluid dynamics the flow of liquid within the helical groove will not readily provide laminar flow. The flow of the liquid within the helical groove will include two components of motion, a primary flow along the groove parallel to the axis of the groove at any point, and it is believed/a secondary flow which is illustrated diagrammatically/in Figure 5. Figure 5 shows a transverse cross section through the groove 101 at right angles to the axis of the groove 101 at that point. It will be seen that/there is a secondary according to our understanding transverse flow of liquid in the groove 101 which is separated into two components one on each side of a radius from the axis 99 of the core 100. As can be seen, the liquid in the groove 101 flows according to this secondary transverse flow pattern from a point 97 in the groove 101

closest to the axis 99 and moves along the radius from the axis 99 towards a point 98 of the groove 101 at a maximum distance from the axis 99. The flow path then separates, one half passing across the membrane 116 in one direction and the other in the other direction and the flow paths then return back to the point 97 along the semi circular walls of the groove 101. The point at which the velocity of the liquid in the secondary flow has its peak is towards the point 98 and this assists in preventing deposition and clotting on the membrane 116.

A further advantage of the described apparatus is that it may readily be disassembled and cleaned. The membrane may be discarded and a fresh membrane used.

Further adaptions of this apparatus allow for the collection of both ions and gases. Two cores 100 may be mounted end to end in a double length cylinder 117, one core 100 being surrounded by a gas permeable membrane 116 and the other surrounded by an ion permeable membrane. Obviously separate bores 118 would be provided in the two halves of the cylinder 117. Furthermore, in place of the straight groove 118 a helical groove corresponding to the helical groove 101 may be provided. If a separate carrier gas or fluid is to be used then inlet and outlet tubes will be provided adjacent the opposite ends of the groove 118. Other similar arrangements will be readily apparent to those skilled in the art.

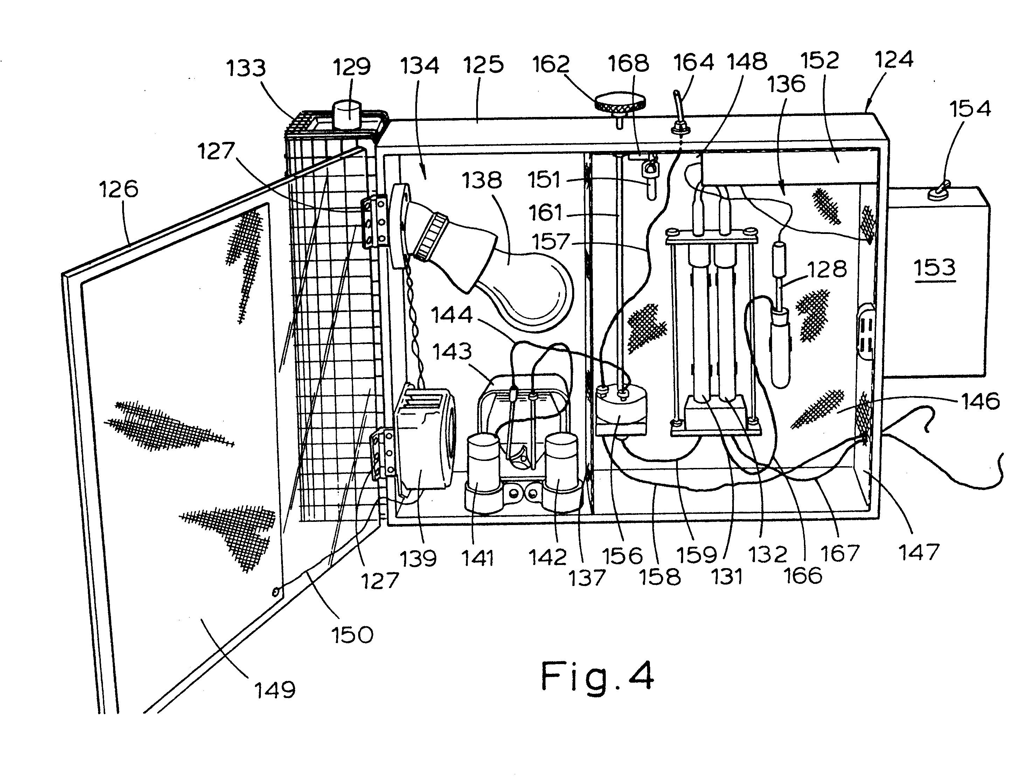

Figure 4 shows a preferred form of electrolyte analysis unit 124 generally corresponding to the electrolyte analysis unit 24 of Figure 1 and including electrode assemblies 128, 131 and 132 corresponding to electrodes 28, 31 and 32 of Figure 1.

The electrolyte analysis unit of Figure 4 comprises a rectangular box 125 in this case made of perspex. As is shown the front of the box comprises a door 126 which in this case is open being hinged at its left hand side by suitable hinges 127. Mounted to the left side of the box 125 is a pre-set thermostat 129 mounted within an earthed cage 133.

The rectangular box 125 is divided into a left hand compartment 134 and a right hand compartment 136, a dividing wall 137 being of metal mesh.

The left hand compartment 134 includes, mounted to the left hand wall a heat supply in the form of a light bulb 138 and an electric motor driven fan 139. Air from the fan 139 passes over the light bulb 138 and is circulated throughout the rectangular box 125. Mounted within the right hand compartment is a

- 20 -

0004760

switch the
thermistor 151 for controlling the thermostat 129 to/
bulb 138 to maintain a constant temperature within
the right hand compartment.

Also mounted within the left hand compartment 134
are two bottles of standard electrolyte 141, 142 the
contents of which may be selectively pumped from the
bottles 141, 142 via a pump 143 to a tube 144.
Selector means to select the bottle from which the
pump 143 pumps liquid is provided (not shown).

As is clear from Figure 4 the right hand compartment
136 is entirely surrounded, when the door 126 is
closed, by metal mesh (other than the floor of the
compartment), the sheet 146 of metal mesh to the back
of the compartment, the sheet 147 to the right of the
compartment, the sheet 148 at the top of the compartment,
and the sheet 149 mounted to the door 126 all being
interconnected by various wires eg wire 150 and being
connected to earth. In this way varying electrostatic
potentials owing to surrounding equipment are isolated
from the electrode assemblies within the right hand
compartment 136.

Electrode assemblies 128, 131 and 132 are mounted in
the right hand compartment 136. The assemblies
include electric wires leading to a box 152 within
the right hand compartment 136 containing the electronic circuits for operating the electrolyte analysis unit 124. The power supply 153 for the electronic box 152 is mounted outside the rectangular box 125 and is provided by means of two 9V batteries mounted within a power supply box and controlled by a switch 154. The electronic circuitry within the electronic box 152 is generally of conventional form and will not be described further.

Also mounted within the right hand compartment 136 is a changeover valve 156 having two input tubes 144, 157 thereto and two output tubes 158, 159 therefrom.

The changeover valve 156 is operated by means of rotation of the valve by a shaft 161 the upper end of which passes out through the upper face of the right hand compartment and to the top of which is mounted a control knob 162. In one position of the changeover valve the input tube 144 is connected to the output tube 159 and the input tube 157 is connected to the output tube 158, and in the other position of the changeover valve 156 the input tube 144 is connected to the output tube 158 and the input tube 157 is connected to the output tube 159. A microswitch 168 is provided attached to the shaft 161 of the changeover valve 156 to switch the pump 143 on and off so that the pump 143 is only switched on when the electrolyte in the bottles 141 or 142 is connected to the electrode assemblies 131, 132.

As is clear from the drawing the input tube 157 passes from the valve 156 up and through a suitable grommet assembly 163 in the upper surface of the right hand compartment 136. It is connected to a tube 164 connected in the present instance directly to the output of the cell 13 (Figure 1) or 113 (Figures 2 and 3). In the present instance there is no pump 25 provided as shown in Figure 1.

The output tube 159 is connected to the electrode assemblies 131 and 132 and a tube 166 interconnects the electrode assemblies 131, 132 with the reference electrode assembly 128. In a preferred arrangement, electrode 131 is adapted to measure the concentration of potassium ions, and electrode 132 is adapted to measure the concentration of calcium ions. An output tube 167 from the electrode assemblies 131, 132 is connected either to waste or to a chamber 39 of the type described with reference to Figure 1. Similarly the tube 158 is connected either to chamber 39 or waste.

The electrolyte analysis unit is used as follows. The lamp 138 is controlled by means of the thermistor 151 so as to maintain a constant temperature within the right hand compartment 136. The electrode assemblies 131 and 132 are initially calibrated by turning the changeover valve 156 and selector means to positions in which electrolyte from the bottle 141 is passed

0004760

by the pump 143 to the electrode assemblies 131 and 132 and their outputs as provided by the electronic circuit 152 are measured. The electrolyte from the second bottle 142 is then passed by means of the pump 143 to the electrode assemblies 131, 132 and a second reading taken.

Changeover valve 156 is then moved to its second position in which electrolyte from the cell 13 or 113 passes along the tube 164 and tube 157, through the changeover valve to the electrode assemblies 131, 132. A reading is then taken and this electrolyte may be continuously monitored as is described with reference to Figure 1. From time to time changeover valve 156 may be moved to its second position to check the calibration of the electrode assemblies.

The present apparatus can therefore be utilised to provide a continuous monitoring of the concentrations of various components of the blood of a patient without allowing that blood to come into contact with complex analysing means such as the various electrodes. It has particular use in the above described mode of operation where it is required in the clinical situation to know when the particular level of a constituent of the blood varies and can provide an immediate indication of this variation whereas hitherto this has not been possible.

- 24 -

0004760

Furthermore, the apparatus can be used with tubing already connected to the patient for the purpose of dialysis, or with canuli inserted in blood vessels. It may be done at the time of dialysis, or when the patient is not connected to the dialyser.

The invention is not restricted to the details of the foregoing example. For example, the cell 13 may be simplified to simply provide a membrane which either passes gas or passes the ions as desired in which case it will only be necessary to have two compartments and one membrane. Alternatively, two separate cells 13 may be provided, one for separating the blood and gas and one for separating the blood and ions.

If necessary the separation of dissolved gases or of water and ions could be effected using several similar cells in series or parallel to obtain an increased membrane area without making the overall dimensions of the unit too great.

Furthermore, other methods of analysing the carrier liquid or the carrier gas may be utilised as are well know. Other means for controlling the flow of the carrier liquid may be provided other than the peristaltic pump 25.

In the cell of Figures 2 and 3 the blood may further be prevented from escaping from between the membrane 116 and the core 100 as follows. An 'O' ring of suitable rubber is provided surrounding the membrane 116 and core 100 at each end. A suitable groove is provided at each end of the inner surface 121 of the cylinder 117 and a suitable lip is provided on the inner face of each plate 110, 111. As the cell is assembled and the plates 110, 111 moved towards one another, they compress the 'O' rings so that they distort towards the core 100 and surrounding membrane thereby firmly clamping the membrane 116 on the core 100.

- 26 -

0004760

## Claims

1. Apparatus for determining the presence of predetermined constituents in a liquid including a helical passage for the liquid, membrane means forming a wall of said helical passage permeable to said predetermined constituents to provide predetermined relative concentrations of said constituents on each side of the membrane and means for analysing a carrier fluid containing the predetermined constituents which have permeated through the membrane.

2. Apparatus as claimed in claim 1 in which the carrier fluid is a separate fluid.

3. Apparatus as claimed in claim 1 in which the carrier fluid is a gas.

4. Apparatus as claimed in claim 1 in which the carrier fluid is liquid.

5. Apparatus as claimed in claim 1 in which the carrier fluid is provided by the liquid.

6. Apparatus as claimed in claim 5 in which the carrier fluid is water separated from the liquid under test.

7.   Apparatus as claimed in any of claims 1 to 6 in which there is provided analysis means for the analysis of the gas or dissolved ions in the carrier fluid, said analysis means including electrodes which are sensitive to the particular constituent in question and a reference electrode.

8.   Apparatus as claimed in any of claims 1 to 7 for determining the presence of predetermined constituents in blood in which means is provided for connecting the apparatus to a patient whereby blood may be continuously withdrawn from a patient, passed through the apparatus and thereafter returned to the patient.

9.   Apparatus as claimed in any of claims 1 to 8 in which the helical passage is formed as a groove on the outside of a cylindrical member.

10. Apparatus as claimed in claim 9 in which the cylindrical member is of silicone rubber.

11. Apparatus as claimed in claim 9 or claim 10 in which the cylindrical member is surrounded by a cylindrical membrane.

12.   Apparatus as claimed in claim 11 in which the membrane is surrounded by an outer sleeve having a groove on its inner surface.

13. Apparatus as claimed in any of claims 1 to 12 in which the means for analysing the carrier fluid includes means for supplying a reference fluid in place of the carrier fluid to calibrate the apparatus.

0004760

Fig.1

0004760

Fig.2

Fig.3

Fig.5

Fig.4

3/3

0004760